# EUROPÄISCHE PATENTANMELDUNG

(11) **EP 1 834 640 A2**
(43) Veröffentlichungstag der Anmeldung: **19.09.2007**
(21) Anmeldenummer: 07111669.3
(22) Anmeldetag: 13.03.2002
(51) Int. Cl.: A61K 31/40

(54) **Kappa-Opiatagonisten für die Behandlung von Erkrankungen der Blase**

(30) Priorität: 05.04.2001 DE 10116978
(62) Teilanmeldung aus: 02724228.8
(71) Anmelder: Tioga Pharmaceuticals, Inc., San Diego, CA 92121 (US)
(72) Erfinder: Jacob, Jutta, 56323, Waldesch (DE); Weber, Frank, 64846, Gross-Zimmern (DE); Bartoszyk, Gerd, 64331, Weiterstadt (DE); Seyfried, Christoph, 64342, Seeheim-Jugenheim (DE)
(74) Vertreter: Viering, Jentschura & Partner

(57) **Zusammenfassung**

Die Erfindung betrifft die Verwendung des Arzneimittels N-Methyf-N-[(1S)-1-phenyl-2-((3S)-3-hydroxypyrrolidin-1-yl)-ethyl]-2,2-diphenyl-acetamid oder eines seiner pharmakofogisch unbedenklichen Salze zur Herstellung von Arzneimittelformulierungen zur Behandlung von Erkrankungen der Blase, insbesondere der Reizblase und der damit verbundenen Schmerzen.

## Beschreibung

Die Erfindung betrifft die Verwendung des Arzneimittels N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Herstellung von Arzneimittelformulierungen zur Behandlung von Erkrankungen der Blase, insbesondere der Reizblase und der damit verbundenen Schmerzen.

Der Arzneimittelwirkstoff N-Methyl-N-[(1S)-1-phenyl-2-((3S)-3-hydroxypyrrolidin-1-yl)-ethyl]-2,2-diphenyl-acetamid (Asimadolin) 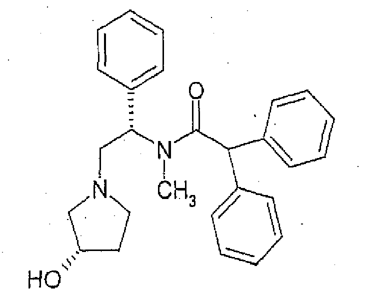 seine pharmakologisch unbedenklichen Salze und ein Verfahren zur Herstellung ist in US 5,532,266 (Beispiel 1) beschrieben.
Der Arzneimittelwirkstoff N-Methyl-N-[(1S)-1-phenyl-2-((3S)-3-hydroxypyrrolidin-1-yl)-ethyl]-2,2-diphenyl-acetamid, insbesondere dessen Hydrochlorid, wirkt analgetisch, antiinflammatorisch, antiasthmatisch, diuretisch, antikonvulsiv, neuroprotektiv und antitussuiv und ist als Kappa-Opiatagonist insbesondere geeignet für die Behandlung einer entzündungsbedingten Hyperalgesie, zur Behandlung von Hirnödemen, bei Unterversorgungszuständen (Hypoxie), Schmerzzuständen, sowie zur Minderung der Folgeschäden einer Ischämie.

Die Verwendung von N-Methyl-N-[(1S)-1-phenyl-2-((3S)-3-hydroxypyrrolidin-1-yl)-ethyl]-2,2-diphenyl-acetamid oder dessen pharmakologisch unbedenklichen Salzen zur Herstellung eines Arzneimittels zur Behandlung von entzündlichen Darmerkrankungen und der damit verbundenen Krankheitssymptome, zur Behandlung von starken Schmerzen, insbesondere von Schmerzübbrempfindlichkeiten auftretend bei Rückenleiden, Brandverletzungen, Sonnenbrand und rheumatischen Erkrankungen sowie zur Behandlung von postoperativen Schmerzen sowie des häufig nach Abdominaloperationen auftretenden Ileus sind in der EP 0 752 246 offenbart.

N-Methyl-N-[(1S)-1-phenyl-2-((3S)-3-hydroxypyrrolidin-1-yl)-ethyl]-2,2 diphenyl-acetamid oder eines der pharmakologisch unbedenklichen Salze ist ebenfalls geeignet zur Behandlung von funktionellen Magen-DarmErkrankungen, die mit Schmerzen und/oder einer vermehrten oder verminderten Perestaltik einhergehen, insbesondere des Irritable Bowel Syndroms oder zur Behandlung der nicht mit einem Ulkus verbundenen Dyspepsie, Obstipation, insbesondere opiod-induzierte Obstipation, von Arthritis, Migräne, Psoriasis oder anderen juckenden Hauterkrankungen, Dysmenorrhoe und Fibromylagia.

Es war Aufgabe der Erfindung, eine pharmazeutisch wirksame Verbindung zur Verfügung zu stellen, die in der Behandlung und/oder Prophylaxe von Erkrankungen der Blase, insbesondere der Reizblase, auch irritable bladder, Zytalgi, Zystalgie, Neuralgia vesicae oder Blasenneurose genannt, einsetzbar und wirksam sind, die gleichzeitig die mit dieser Erkrankung verbundenen Schmerzen lindern und die Erkrankung heilen.

Der Begriff Reizblase steht für einen vor allem bei Frauen vorkommenden chronischen Reizzustand des unteren Harntrakts. Symptome sind Dysurie, imperativer Harndrang, Pollakisurie, suprapubische und diffuse Schmerzen beim Sitzen. Häufig besteht eine ausgeprägte Diskrepanz zwischen subjektiven Beschwerden und den objektiven Befunden. Häufigste Ursachen sind Störungen des psychovegetativen oder endokrinen Systems. Die Reizblase ist zu unterscheiden von anderen Krankheitsbildern, wie Harnweginfektionen und Veränderungen des unteren Harntrakts, Erkrankungen benachbarter Beckenorgane oder ZNS-oder Rückenmarkerkrankungen (z.B. Multiple Sklerose).

Es wurde überraschenderweise gefunden, daß der Arzneimiftelwirkstoff N-Methyl-N-[(1S)-1-phenyl-2-((3S)-3-hydroxypyrrolidin-1-yl)-ethyl]-2,2-diphenyl-acetamid oder eines der pharmakologisch unbedenklichen Salze, insbesondere das Hydrochlorid überraschend wirksam ist zur Behandlung von Erkrankungen der Blase, insbesondere der Reizblase, trotz der bekannten diuretischen Wirkung von Asimadolin.

Gegenstand der Erfindung ist daher die Verwendung des Arzneimittels N-Methyl-N-[(1-S)-1-phenyl-2-((3S)-3-hydroxypyrrolidin-1-yl)-ethyl]-2,2 diphenyl-acetamid oder eines seiner pharmakologisch unbedenklichen Salze zur Herstellung von Arzneimittelformulierungen zur Behandlung von Erkrankungen der Blase.

Gegenstand der Erfindung ist daher insbesondere die Verwendung des Arzneimittels N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Herstellung von Arzneimittelformulierungen zur Behandlung der Reizblase.

N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze kann daher zur Herstellung pharmazeutischer Präparate zur Behandlung von Erkrankungen der Blase, insbesondere der Reizblase, verwendet werden, indem man sie zusammen mit mindestens einem Träger- oder Hilfsstoff und, falls erwünscht, mit einem oder mehreren weiteren Wirkstoffen in die geeignete Dosierungsform bringt.

Gegenstand der Erfindung ist daher auch eine pharmazeutische Zubereitung, gekennzeichnet durch einen Gehalt an N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Behandlung von Erkrankungen der Blase.

Gegenstand der Erfindung ist daher insbesondere auch eine pharmazeutische Zubereitung, gekennzeichnet durch einen Gehalt an N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Behandlung der Reizblase.

Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale(z. B. orale oder rektale) oder parenterale Applikation eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk oder Cellulose.

Zur oralen Anwendung dienen insbesondere Tabletten, Dragees, Kapseln, Sirupe, Säfte oder Tropfen. Von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wäßrige Lösungen, ferner Suspensionen, Emulsionen oder Implantate.

Die erfindungsgemäß beanspruchten Wirkstoffe können auch lyophilisiert und die erhaltenen Lyophilisate z. B. zur Herstellung von injektionspräparaten verwendet werden.

Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine,

N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze wird in der Regel in Analogie zu anderen bekannten, für die beanspruchte Indikation im Handel erhältlichen Präparaten verabreicht, vorzugsweise in Dosierungen zwischen etwa 0,1 mg und 50 mg, insbesondere zwischen 5 und 30 mg pro Dosierungseinheit. Die tägliche Dosierung liegt vorzugsweise zwischen etwa 0.02 und 20 mg/kg, insbesondere 0,1 und 10 mg/kg Körpergewicht.

Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Im Folgenden werden Tiermodelle beschrieben, die die Wirksamkeit von Asimadolin für die Behandlung von Erkrankungen der Blase, insbesondere der Reizblase belegen.

Ein Modell zur Messung des Einflusses auf die Urinausscheidung ist bei Lipschitz et. al., J. Pharmacol. Exp. Ther. 1943; 79: 97-110 beschrieben. Die zu untersuchende Substanz wird Ratten gegeben, denen vorher über Nacht das Futter entzogen wurde bei freiem Wasserzugang. Eine erhöhte Urinauscheidung wird provoziert durch die gleichzeitige intraperitonelae Injektion von 100 ml/kg physiologischer Kochsalzlösung. Unmittelbar nach Substanzgabe wurde die Harnblase durch leichtes Massieren des Abdomens über der Harnblase entleert. Anschliessend werden die Ratten in Metabolismuskäfigen gehalten, in denen der Urin über den Zeitraum von 6 Stunden aufgefangen wird. N-Methyl-N-[(1S)-1-phenyl-2-((3S)-3-hydroxypyrrolidin-1-yl)-ethyl]-2,2-diphenyl-acetamid erhöht die Urinausscheidung dosisabhängig, wobei eine 2fach höhere Urinmenge ausgeschieden wird bei der Dosis von 100 mg/kg.

In Analogie wird die Wirkung auf die Urinausscheidung bei normalen Ratten geprüft (d.h. ohne die Induktion einer erhöhten Urinausscheidung, s.o.). N-Methyl-N-[(1S)-1-phenyl-2-((3S)-3-hydroxypyrrolidin-1-yl)-ethyl]-2,2-diphenyl-acetamid erhöht die Urinausscheidung dosisabhängig, wobei bereits bei 30 mg/kg po eine 5,5fach höhere Urinmenge ausgeschieden wird.

Das klassiche Tiermodell für die Reizblase ist beschrieben bei Ghoniem et al., Neurourol. Urodyn. 1995; 14: 657-65. Bei weiblichen Affen wird eine Reizblase durch die direkte Infusion von Azeton in die Blase induziert. Die Tiere werden in Matabolismuskäfigen gehalten, die für das kontinuierliche Monitoring der Miktion (Harnlassen) der Tiere ausgelegt sind. Über Harnflussmesser werden Frequenz, Entleerungsvolumen und Flussgeschwindigkeit des Harns kontinuierlich gemessen. Der Vergleich der Harnstoffabsorption vor und nach der Azetoninfusion zeigt, dass die Harnstoffabsorption nach Azetoninfusion drastisch erhöht ist und erst nach vier Wochen wieder den Basiswert vor der Azetoninfusion erreicht. Ferner werden in der ersten Woche nach Azetoninfusion starke Veränderungen der Blasenphysiologie beobachtet: Die Blasenleistung, gemessen in ml/cm, erniedrigt sich um fast 95%. Auch das Entleerungsverhalten ändert sich stark, wobei die Frequenz der Entleerungen stark ansteigt mit dem Bild eines häufigen Tröpfelns und dabei mit einem um ca. 70% verminderten Entleerungsvolumen. Die systematische Verhaltensbeobachtung der Tiere über vier Wochen ergibt als Verhaltensrepertoire eine erniedrigte Frequenz allgemeiner und insbesondere sozialer Aktivitäten, während stereotype, auf sich selbst bezogene Verhaltensweisen wie sich Lausen, Kratzen, Kraulen stark zunehmen. Diese bei den Affen beobachteten Verhaltensänderungen sind konsistent mit dem klinischen Bild erheblicher Beschwerden und Schmerzen. N-Methyl-N-[(1S)-1-phenyl-2-((3S)-3-hydroxypyrrolidin-1-yl)-ethyl]-2,2-diphenyl-acetamid in Dosierungen von 3, 10, und 30 mg/kg normalisiert die Blasenfunktion dosisabhängig.

## Patentansprüche

1. Verwendung des Arzneimittelwirkstoffs N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Herstellung eines Arzneimittels zur Behandlung von Erkrankungen der Blase.

2. Pharmazeutische Zubereitung, **gekennzeichnet durch** einen Gehalt an mindestens N-Methyl-N- [(1S) -1-phenyl-2- ((3S) -3-hydroxypyrrolidin-1yl)-ethyl]-2,2-diphenyl-acetamid oder einem der physiologisch unbedenklichen Salze zur Behandlung von Erkrankungen der Blase.

3. Verwendung des Arzneimittelwirkstoffs N-Methyl-N-[(1S)-1-phenyl-2-((3S)-3-hydroxypyrrolidin-1-yl)-ethyl]-2,2-diphenyl-acetamid oder eines seiner pharmakologisch unbedenklichen Salze zur Herstellung eines Arzneimittels zur Behandlung der Reizblase.

4. Pharmazeutische Zubereitung, **gekennzeichnet durch** einen Gehalt an mindestens N-Methyl-N-[(1S)-1-phenyl-2-((3S)-3-hydroxypyrrolidin-1-yl)-ethyl]-2,2-diphenyl-acetamid oder einem der physiologisch unbedenklichen Salze zur Behandlung und/oder Prophylaxe der Reizblase.

5. N-Methyl-N-[(1S)-1-phenyl-2-((3S)-3-hydroxypyrrolidin-1-yl)-ethyl]-2,2-diphenyl-acetamid oder eines seiner unbedenklichen Salze für die Verwendung zur Behandlung von Erkrankungen der Blase.

6. Die Verbindung gemäß Anspruch 5, wobei die Verbindung N-Methyl-N-[(1S)-1-phenyl-2-((3S)-3-hydroxypyrrolidin-1-yl)-ethyl]-2,2-diphenyl-acetamid-hydrochlorid ist.

7. Die Verbindung gemäß Anspruch 5 oder 6, wobei die Erkrankung der Blase eine Reizblase ist.

8. Die Verbindung gemäß einem der Ansprüche 5 bis 7, wobei die Behandlung die Blasenfunktion normalisiert.

9. Die Verbindung gemäß einem der Ansprüche 5 bis 7, wobei die Behandlung ein oder mehrere Symptome der Reizblase, die aus der Gruppe ausgewählt werden, die aus Dysurie, imperativem Harndrang, Pollakisurie, suprapubischen und diffusen Schmerzen beim Sitzen besteht, lindern.

10. Die Verbindung gemäß einem der Ansprüche 5 bis 7, wobei. N-Methyl-N-[(1S)-1-phenyl-2-((3S)-3-hydroxypyrrolidin-1-yl)-ethyl]-2,2-diphenyl-acetamid oder eines seiner unbedenklichen Salze in einer Dosierung zwischen etwa 0,1 mg und 50 mg, insbesondere zwischen 5 mg und 30 mg pro Dosierungseinheit verabreicht wird.

11. Die Verbindung gemäß einem der Ansprüche 5 bis 7, wobei N-Methyl-N-[(1S)-1-phenyl-2-((3S)-3-hydroxypyrrolidin-1-yl)-ethyl]-2,2-diphenyl-acetamid oder eines seiner unbedenklichen Salze in einer täglichen Dosierung zwischen etwa 0,02 mg/kg und 20 mg/kg, insbesondere zwischen 0,1 mg/kg und 10 mg/kg Körpergewicht verabreicht wird.

12. Ein Verfahren zum Herstellen eines Medikamentes zur Behandlung von Erkrankungen der Blase oder der Reizblase, **dadurch gekennzeichnet, dass** der Arzneimittelwirkstoff N-Methyl-N-[(1S)-1-phenyl-2-((3S)-3-hydroxypyrrolidin-1-yl)-ethyl]-2,2-diphenyl-acetamid oder eines seiner unbedenklichen Salze zusammen mit mindestens einem Träger- oder Hilfsstoff und wahlweise einem oder mehreren weiteren Wirkstoffen in die geeignete Dosierungsform gebracht wird.

13. Das Verfahren gemäß Anspruch 12, wobei der Arzneimittelwirkstoff N-Methyl-N-[(1S)-1-phenyl-2-((3S)-3-hydroxypyrrolidin-1-yl)-ethyl]-2,2-diphenyl-acetamid-hydrochlorid ist.
